# EUROPÄISCHE PATENTSCHRIFT

(11) **EP 1 791 805 B1**
(45) Veröffentlichungstag und Bekanntmachung des Hinweises auf die Patenterteilung: **12.10.2011**
(21) Anmeldenummer: 05774100.1
(22) Anmeldetag: 10.08.2005
(51) Int. Cl.: C07C 213/10, C07C 215/12

(54) **VERFAHREN ZUR HERSTELLUNG VON TRIETHANOLAMIN**
METHOD FOR PRODUCING TRIETHANOLAMINE
PROCEDE POUR PRODUIRE DE LA TRIETHANOLAMINE

(30) Priorität: 09.09.2004 DE 102004044091
(43) Veröffentlichungstag der Anmeldung: 06.06.2007
(73) Patentinhaber: BASF SE, 67056 Ludwigshafen (DE)
(72) Erfinder: HAESE, Frank, 24855 Bollingstedt (DE); MELDER, Johann-Peter, 67459 Böhl-Iggelheim (DE); DOSTALEK, Roman, 67271 Neuleiningen (DE); JULIUS, Manfred, 67117 Limburgerhof (DE)
(86) Internationale Anmeldenummer: PCT/EP2005/008679
(87) Internationale Veröffentlichungsnummer: WO 2006/027077

(56) Entgegenhaltungen:
- EP-A- 0 004 015
- EP-A- 1 132 371
- US-A- 3 207 790
- MOSHER, EARL ET AL: "The chemical control of phosphine gas generation during the machining of nodular cast iron" LUBRICATION ENGINEERING , 45(7), 445-50 CODEN: LUENAG; ISSN: 0024-7154, 1989, XP008053530
- Dräger: "Brochure: Draeger-Tubes and accuro Pump", pages 1-6,

## Beschreibung

Die vorliegende Erfindung betrifft ein Verfahren zur Herstellung von Triethanolamin und Triethanolamin enthaltend eine oder mehrere bestimmte phosphorhaltige Verbindungen.

Wichtige Einsatzgebiete von Triethanolamin (TEOA) oder dessen Folgeprodukten sind beispielsweise Seifen, Waschmittel und Shampoos in der kosmetischen Industrie oder auch Dispergiermittel und Emulgiermittel.

Für diese und andere Einsatzgebiete ist wasserklares, farbloses Triethanolamin mit einer möglichst geringen Verfärbung, z.B. gemessen als APHA- oder Gardner-Farbzahl, das diese Eigenschaften auch über längere Lagerzeiten (von z.B. 6, 12 oder mehr Monaten) beibehält, erwünscht.

Ein bekanntes Problem ist, dass ein nach einer fraktionierenden Destillation eines Triethanolamin-Rohprodukts, das z.B. durch Umsetzung von Ammoniak mit Ethylenoxid gewonnen wurde, erhaltenes reines TEOA eine gelbliche bis bräunliche oder rosa Verfärbung aufweist (Farbzahl z.B. ca. 10 bis 500 APHA nach DIN ISO 6271(= Hazen)). Diese Verfärbung tritt besonders in Prozessen auf, in denen hohe Temperaturen durchlaufen werden.

Bei einer Lagerung des Alkanolamins, auch im geschlossenen Gebinde und unter Lichtausschluss, wird diese Verfärbung noch weiter verstärkt. (Siehe z.B.: T.I. MacMillan, Ethylene Oxide Derivatives, Report No. 193, Kapitel 6, Seiten 6-5 und 6-9 bis 6-13, 1991, SRI International, Menlo Park, California 94025;
G.G. Smirnova et al., J. of Applied Chemistry of the USSR 61, S. 1508-9 (1988), und Chemical 8 Engineering News 1996, Sept. 16, Seite 42, mittlere Spalte).

In der Literatur sind verschiedene Verfahren zur Herstellung von Triethanolamin mit verbesserter Farbqualität beschrieben.

EP-A-36 152 und EP-A-4015 (beide BASF AG) erläutern den Einfluss der in Verfahren zur Herstellung von Alkanolaminen eingesetzten Werkstoffe auf die farbliche Qualität der Verfahrensprodukte und empfehlen nickelfreie bzw. nickelarme Stähle.

US-A-3,207,790 (Dow Chemical Company) beschreibt ein Verfahren zur Verbesserung der Farbqualität von Alkanolaminen durch Zugabe eines Borhydrids eines Alkalimetalls.

EP-A-1 081 130 (BASF AG) betrifft ein Verfahren zur Herstellung von Alkanolaminen mit verbesserter Farbqualität durch Behandlung des Alkanolamins mit Wasserstoff in Gegenwart eines Hydrierkatalysators.

EP-A-4015 (BASF AG) beschreibt, dass Mono-, Di- und Triethanolamin mit geringerer Verfärbung durch Zusatz von phosphoriger oder unterphosphoriger Säure oder deren Derivate vor oder während oder direkt nach der stufenweisen Umsetzung von Ethylenoxid mit Ammoniak und anschließende Isolierung durch Destillation erhalten werden.

WO-A-00/32553 (BASF AG) betrifft ein Verfahren zur Reinigung von TEOA, hergestellt durch Umsetzung von wässrigem Ammoniak mit Ethylenoxid in flüssiger Phase unter Druck und bei erhöhter Temperatur, indem man vom Umsetzungsprodukt überschüssiges Ammoniak, Wasser und Monoethanolamin abtrennt, das so erhaltene Rohprodukt mit Ethylenoxid umsetzt und anschließend in Gegenwart von phosphoriger oder unterphosphoriger Säure oder deren Verbindungen rektifiziert.

EP-A-1 132 371 (BASF AG) betrifft ein Verfahren zur Herstellung von Alkanolaminen mit verbesserter Farbqualität, wobei man das Alkanolamin mit einer wirksamen Menge von phosphoriger oder unterphosphoriger Säure oder deren Verbindungen zunächst bei erhöhter Temperatur über einen Zeitraum von mindestens 5 Min. behandelt (Schritt a) und anschließend in Gegenwart einer wirksamen Menge einer dieser Phosphorverbindungen destilliert (Schritt b).

Die ältere deutsche Patentanmeldung Nr. 102004042453.5 vom 31.08.04 (BASF AG) betrifft ein Verfahren zur Herstellung von Triethanolamin, wobei man dem Triethanolamin phosphorige und/oder unterphosphorige Säure und eine basische Verbindung, ausgewählt aus Alkalimetallhydroxid, Erdalkalimetallhydroxid und [R¹R²R³(2-hydroxyethyl)ammonium]hydroxid, wobei R¹, R² und R³ unabhängig voneinander C₁₋₃₀-Alkyl oder C₂₋₁₀-Hydroxyalkyl bedeuten, zusetzt und im Fall von Alkalimetallhydroxid als basischer Verbindung das Mol-Verhältnis Säure(n) : Hydroxid im Bereich von 1 : 0,1 bis 1 : 1 liegt und im Fall von Erdalkalimetallhydroxid als basischer Verbindung das Mol-Verhältnis Säure(n) : Hydroxid im Bereich von 1 : 0,05 bis 1 : 0,5 liegt.

Der vorliegenden Erfindung lag die Aufgabe zugrunde, ein gegenüber dem Stand der Technik verbessertes Verfahren zur Herstellung von Triethanolamin mit hoher Farbqualität bereitzustellen. Das Verfahren soll die Verfärbung von TEOA, z.B. gemessen als APHA-Farbzahl, vermindern und die Farbstabilität verbessern (unerwünschte Zunahme der Farbzahl über die Lagerzeit). Insbesondere sollte das Verfahren gegenüber den Verfahren der Anmeldungen EP-A-4015, WO-A-00/32553 und EP-A-1 132 371 höhere Ausbeuten an TEOA liefern.

Demgemäß wurde ein Verfahren zur Herstellung von Triethanolamin gefunden, welches dadurch gekennzeichnet ist, dass man dem Triethanolamin ein Phosphan und/oder eine Verbindung, die ein Phosphan freisetzt, zusetzt.

Weiterhin wurde Triethanolamin enthaltend ein Phosphan und/oder eine Verbindung, die ein Phosphan freisetzt, gefunden.

Bevorzugte Ausführungsformen des Verfahrens und des Triethanolamins sind den Unteransprüchen 2 bis 13 und 15 bis 21 zu entnehmen.

Erfindungsgemäß wurde erkannt, dass unter Beibehaltung oder sogar Verbesserung der Farbqualität gegenüber den Verfahren des Stands der Technik, durch den erfindungsgemäßen Zusatz an Phosphorverbindung(en) die Bildung von Nebenprodukten im TEOA erheblich vermindert wird. Gleichzeitig wird die TEOA-Destillationsausbeute gesteigert. Die verminderte Nebenproduktbildung beruht vermutlich auf der nicht sauren bzw. weniger sauren Wirkung der Phosphorverbindungen, d.h. des Phosphans und/oder der Verbindung, die ein Phosphan freisetzt.

Wird ein Phosphan zugesetzt, treten vorteilhaft keine Probleme durch Salzbildung auf.

Darüber hinaus können gegenüber den bekannten Verfahren unter Verwendung von phosphoriger oder unterphosphoriger Säure oder deren Verbindungen als Zusatz bei gleicher Wirkung geringere Mengen an Phosphan und/oder Verbindung, die ein Phosphan freisetzt, eingesetzt werden.

Das im erfindungsgemäßen Verfahren eingesetzte Triethanolamin kann nach bekannten Verfahren, insbesondere durch Umsetzung von Ammoniak mit Ethylenoxid (z.B. gemäß EP-A-673 920 oder WO-A-00132553) erhalten werden.

Die Reinheit des im erfindungsgemäßen Verfahren eingesetzten Triethanolamins beträgt bevorzugt größer 70 Gew.-%, insbesondere größer 80 Gew.-%. Neben destilliertem oder undestilliertem rohen Triethanolamin, das auch direkt in roher Form einer Anlage zur Herstellung des Alkanolamins aus den entsprechenden Vorstufen entnommen werden kann, kann auch destilliertes TEOA mit einer Reinheit von größer 90 Gew.%, z.B. größer 95 Gew.-%, besonders 97 Gew.-%, insbesondere ≥ 98 Gew.-%, ganz besonders ≥ 99 Gew.-%, eingesetzt werden.

Es können auch Mischungen von Triethanolamin mit anderen Alkanolaminen, wie z.B. Monoethanolamin (MEA), Diethanolamin (DEA), Aminodiglykol (ADG, H₂NCH₂CH₂OCH₂CH₂OH), O,N,N-Tris-(2-hydroxyethyl)-ethanolamin, N-(2-Aminoethyl)-ethanolamin (AEEA), N-(2-Hydroxyethyl)-piperazin, N-(2-Hydroxyethyl)-morpholin, N,N'-Bis-(2-Hydroxyethyl)-piperazin, Monoisopropanolamin, Diisopropanolamin, Triisopropanolamin und 1 ,3-Propanolamin, oder Lösungen von Triethanolamin in einem inerten Lösungsmittel, wie z.B. Alkohole (Methanol, Ethanol, iso-Propanol, n-Propanol, n-Butanol, 2-Ethylhexanol), Ether (Tetrahydrofuran, 1,4-Dioxan), Kohlenwasserstoffe (Benzol, Pentan, Petrolether, Toluol, Xylol, Hexan, Heptan, Mihagol) und Wasser oder Mischungen hiervon, eingesetzt werden.

Die APHA-Farbzahl des eingesetzten Triethanolamins beträgt bevorzugte 100, insbesondere ≤ 50, ganz besonders ≤ 20, z.B. 2 bis 15.

Das erfindungsgemäße Verfahren lässt sich wie folgt ausführen.

Das in seiner Farbqualität zu verbessernde Triethanolamin in flüssiger Phase, optional in Gegenwart eines inerten Lösungsmittels, wird in einem geeigneten Behälter, z.B. Rührbehälter, der mit einem Rückflusskühler ausgerüstet sein kann, mit einer wirksamen Menge an Phosphan und/oder einer Verbindung, die ein Phosphan freisetzt, vorteilhaft unter Rühren oder Umpumpen, versetzt.
Gasförmiges PH₃ kann z.B. über ein Einleitungsrohr in das TEOA eingebracht werden.

Die Mischung wird über einen Zeitraum von bevorzugt mindestens 5 Min., insbesondere mindestens 10 Min. (beispielsweise 10 Min. bis 50 Stunden, insbesondere 10 Min. bis 24 Stunden), ganz besonders mindestens 15 Min. (beispielsweise 15 Min. bis 6 Stunden), besonders bevorzugt mindestens 30 Min. (beispielsweise 30 Min. bis 4 Stunden oder 40 Min. bis 3 Stunden oder 60 Min. bis 2 Stunden), auf eine Temperatur im Bereich von 40 bis 250°C, insbesondere 70 bis 240°C, ganz besonders 100 bis 230°C, besonders bevorzugt 120 bis 220°C, z.B. 150 bis 200°C, erwärmt.

Vorteilhaft liegen die Temperaturen bevorzugt niedriger als bei Verwendung von phosphoriger oder unterphosphoriger Säure oder deren Verbindungen als Zusatz gemäß dem Stand der Technik.

Bei dem Phosphan, welches direkt zugesetzt und/oder aus der zugesetzten Verbindung (vide infra) freigesetzt wird, handelt es sich um eine Phosphorwasserstoffverbindung.

Die Phosphane besitzen bevorzugt die allgemeine Formel PₙHₙ₋₂, mit n = 1 bis 9, bevorzugt n = 1, 2, 3 oder 4, oder PₘHₘ, mit m = 1 bis 9.

Bei dem Phosphan, welches entweder direkt zugesetzt und/oder aus der zugesetzten Verbindung freigesetzt wird, handelt es sich besonders bevorzugt um PH₃ (Phosphorwasserstoff), P₂H₄ (Diphosphan) oder P₄H₆ (Tetraphosphan), insbesondere um PH₃. Die Phosphane sind nach bekannten Methoden herstellbar (vgl. z.B. Römpp ChemieLexikon, 10. Aufl., Band 4, Seite 3275-6 und dort zitierte Literatur) und teilweise auch kommerziell erhältlich, wie z.B. PH₃.

Bei der phosphorhaltigen Verbindung, die ein Phosphan freisetzt, handelt es sich z.B. um ein Phosphoniumhalogenid, PH₄X, mit X = Cl, Br oder I.

Bevorzugt handelt es sich bei der Verbindung, die ein Phosphan freisetzt, um ein Phosphid, insbesondere ein Metallphosphid. In diesen Verbindungen besitzt der Phosphor die Oxidationsstufe -III.

Bei dem Metall des Metallphosphids ist ein Metall der Gruppen IA, IIA, IIIA oder IIB des Periodensystems der Elemente bevorzugt.

Insbesondere handelt es sich bei dem Metallphosphid um Lithiumphosphid (Li₃P), Natriumphosphid (Na₃P), Kaliumphosphid (K₃P), Magnesiumphosphid (Mg₃P₂), Calciumphosphid (Ca₃P₂), Aluminiumphosphid (AlP), Indiumphosphid (InP) oder Zinkphosphid (Zn₃P₂).

Die Freisetzung von PH₃ aus Metallphosphiden wird z.B. in

H.-G. von Schnering in A.L. Rheingold, 'Homoatomic Rings, Chains, and Macromolecules of Main-Group Elements', Elsevier, Amsterdam 1977, Seiten 317-348, M. Dörnemann und H. Reif, DE-A-36 18 297,
R.C. Mariott et al., lnorg. Synth. 1973, 4, Seiten 1-4,
H.W. Hilton, W.H. Robison, J. Agric. Food Chem., 1972, 20, Seiten 1209-1213, und US-A-3,387.934 (12.10.1964, Hooker Chemical Corp.)
beschrieben.

Die Metallphosphide sind nach bekannten Verfahren erhältlich und teilweise auch kommerziell verfügbar.

Selbstverständlich kann es sich bei der Verbindung, die ein Phosphan freisetzt, auch um ein Verbindungsgemisch, z.B. ein Gemisch aus einem oder mehrerer Metallphosphide, handeln.

Die Menge an zugesetztem Phosphan und/oder zugesetzter Verbindung, die ein Phosphan freisetzt, beträgt mindestens 0,001 Gew.-%, z.B. mindestens 0,01 Gew.-%, insbesondere 0,02 bis 2 Gew.-%, besonders bevorzugt 0,03 bis 1,0 Gew.-%, ganz besonders bevorzugt 0,5 bis 0,9 Gew.-%, bezogen auf die eingesetzte Menge an Triethanolamin (berechnet reines TEOA); die Wirkung tritt jedoch auch bei größeren Mengen ein.
Werden ein Phosphan und eine Verbindung, die ein Phosphan freisetzt, zusammen eingesetzt, beziehen sich die obigen Mengenangaben auf beide Zusätze zusammen.

Für die bessere Handhabbarkeit kann es von Vorteil sein, die wirksame Menge des Phosphans und/oder der Verbindung, die ein Phosphan freisetzt, in einem geeigneten inerten Verdünnungs- oder Lösungsmittel, wie z.B. Wasser, Alkohole (Methanol, Ethanol, iso-Propanol, n-Propanol, n-Butanol, 2-Butanol, Pentanole, Hexanole), Ether (Tetrahydrofuran, 1,4-Dioxan) oder einem Alkanolamin (z.B. einem Ethanolamin, wie Monoethanolamin, Diethanolamin, N-(2-Aminoethyl)-ethanolamin, insbesondere Triethanolamin), in Form einer z.B. 1 bis 40 Gew.-%igen, besonders 5 bis 30 Gew.-%igen, insbesondere 10 bis 20 Gew.-%igen, Lösung oder Suspension, zuzudosieren.

Die erforderliche Behandlungsdauer des Triethanolamins mit dem Zusatz bzw. den Zusätzen ergibt sich unter anderem aus dem Grad der Verfärbung des eingesetzten Triethanolamins und dem Ausmaß der gewünschten Entfärbung und/oder Farbstabilität des TEOAs. Sie ist in der Regel bei gegebener Temperatur umso größer, je höher der Grad der Verfärbung des im erfindungsgemäßen Verfahren eingesetzten Triethanolamins ist und je höher die Anforderungen an die farbliche Qualität des Verfahrensproduktes sind.

Die Temperatur darf jedoch nicht zu hoch, d. h. in der Regel nicht höher als 250°C, gewählt sein, da ansonsten eine Degradation des Triethanolamins eintreten kann, die die Farbqualität des schließlich erhaltenen TEOAs negativ beeinflusst.

Die für das jeweilig eingesetzte Triethanolamin günstigsten Temperaturen und Behandlungsdauern sind in einfachen Vorversuchen leicht zu ermitteln.

Bei dieser Behandlung des Triethanolamins mit dem Phosphan und/oder der Verbindung, die ein Phosphan freisetzt, ist es von Vorteil, wenn die Mischung während der gesamten Behandlungsdauer oder zeitweise weiter durchmischt (z.B. gerührt oder im Kreislauf umgepumpt) wird.

Weiterhin ist es von Vorteil, wenn die Behandlung des Triethanolamins unter einer Schutzgasatmosphäre (z.B. N₂ oder Ar), also in Abwesenheit von O₂. durchgeführt wird.

Die Behandlung des Alkanolamins mit dem Phosphan und/oder der Verbindung, die ein Phosphan freisetzt, kann auch kontinuierlich in geeigneten Behältern, z.B. in einem Rohrreaktor oder in einer Rührbehälterkaskade, durchgeführt werden.

Die Behandlung des Triethanolamins mit dem Phosphan und/oder der Verbindung, die ein Phosphan freisetzt, kann vorteilhaft im Sumpfbehälter einer Destillationskolonne oder in einem Destillationsvorlagegefäß vor und/oder während der Destillation des Triethanolamins durchgeführt werden.

In einer besonderen Ausgestaltung wird während der Behandlung des Triethanolamins mit dem Phosphan und/oder der Verbindung, die ein Phosphan freisetzt, ein Inertgas (z.B. N₂ oder Ar) als Strippstrom durch das Triethanolamin geleitet, um entstehende Leichtsieder, die sich negativ auf die Farbqualität auswirken können, wie z.B. Acetaldehyd oder dessen Folgeprodukte, aus dem Gemisch zu entfernen.

In einer anderen besonderen Ausgestaltung wird das zu behandelnde Triethanolamin im Kreislauf flüssig über einen Wärmetauscher geführt und werden entstehende Leichtsieder, die sich negativ auf die Farbqualität auswirken können, wie z.B. Acetaldehyd, dabei abgeführt.
Bei dem Wärmetauscher kann es sich hierbei um einen offenen Wärmetauscher, wie z.B. einen Fallfilm- oder Wischblattverdampfer, oder einen geschlossenen Wärmetauscher, wie z.B. einen Platten- oder Rohrbündelwärmetauscher, handeln.

Je nach den gewählten Reaktionsbedingungen kann es notwendig sein, die Behandlung des Triethanolamins mit dem Phosphan und/oder der Verbindung, die ein Phosphan freisetzt, bei einem Überdruck (z.B. 0,1 bis 50 bar) durchzuführen, um das unerwünschte Entweichen einer oder mehrerer Komponenten aus der Mischung zu vermeiden.

In einer besonders bevorzugten Ausführungsform des erfindungsgemäßen Verfahrens wird die Behandlung des Triethanolamins mit dem Phosphan und/oder der Verbindung, die ein Phosphan freisetzt, in Gegenwart von Wasser durchgeführt, insbesondere dann, wenn als Verbindung, die ein Phosphan freisetzt, ein Metallphosphid eingesetzt wird.
Der Wassergehalt des Triethanolamins liegt bevorzugt im Bereich von 0,01 bis 20 Gew.-%, insbesondere im Bereich von 0,02 bis 10 Gew.-%, ganz besonders im Bereich von 0,03 bis 5 Gew.-%, wie z.B. im Bereich von 0,04 bis 2 Gew.-%, jeweils bezogen auf das reine Triethanolamin.
Das Wasser wird dem im erfindungsgemäßen Verfahren eingesetzten TEOA zugesetzt oder/und das TEOA ist bereits wasserhaltig, z.B. bedingt durch die vorangegangene TEOA-Herstellstufe.

Die Destillation oder Rektifikation des Triethanolamins zur Abtrennung des Zusatzes oder der Zusätze erfolgt diskontinuierlich oder kontinuierlich bei einem Druck von in der Regel kleiner 100 mbar (100 hPa), beispielsweise bei ca. 10 bis 50 mbar oder 1 bis 20 mbar, bevorzugt bei 0,5 bis 5 mbar, und bei Sumpftemperaturen von in der Regel 100 bis 250°C, wobei bei der kontinuierlichen Fahrweise in einer besonderen Ausgestaltung gegebenenfalls vorhandene Leichtsiederanteile über Kopf abgezogen werden und man das TEOA im Seitenabzug erhält.

Der die zugesetzte/n Verbindung/en und/oder deren Umsetzungsprodukte enthaltende Rückstand der Destillation oder Rektifikation kann in einer besonderen Ausführungsform vollständig oder teilweise in das Destillationsverfahren zurückgeführt werden.

Das erfindungsgemäße Verfahren liefert ein in der Farbqualität verbessertes Triethanolamin, das direkt nach seinem Erhalt eine APHA-Farbzahl im Bereich von 0 bis 30, insbesondere von 0 bis 20, ganz besonders von 0 bis 10, z.B. 1 bis 6, aufweist.

Alle APHA-Angaben in diesem Dokument erfolgen nach DIN ISO 6271(= Hazen).
Alle ppm-Angaben in diesem Dokument beziehen sich auf das Gewicht (Gew.-ppm).

## Patentansprüche

1. Verfahren zur Herstellung von Triethanolamin, **dadurch gekennzeichnet, dass** man dem Triethanolamin ein Phosphan und/oder eine Verbindung, die ein Phosphan freisetzt, zusetzt.

2. Verfahren nach Anspruch 1, **dadurch gekennzeichnet, dass** es sich bei dem Phosphan um PH₃, P₂H₄ oder P₄H₆ handelt.

3. Verfahren nach den Ansprüchen 1 oder 2, **dadurch gekennzeichnet, dass** es sich bei der Verbindung, die ein Phosphan freisetzt, um ein Metallphosphid handelt.

4. Verfahren nach einem der vorhergehenden Ansprüche, **dadurch gekennzeichnet, dass** man dem Triethanolamin das Phosphan und/oder die Verbindung, die ein Phosphan freisetzt, vor und/oder während der Destillation des Triethanolamins zusetzt.

5. Verfahren nach einem der vorhergehenden Ansprüche, **dadurch gekennzeichnet, dass** man das Triethanolamin mit dem Zusatz über einen Zeitraum von mindestens 5 Min. behandelt.

6. Verfahren nach einem der Ansprüche 1 bis 4, **dadurch gekennzeichnet, dass** man das Triethanolamin mit dem Zusatz über einen Zeitraum im Bereich von 10 Min. bis 50 Stunden behandelt.

7. Verfahren nach einem der beiden vorhergehenden Ansprüche, **dadurch gekennzeichnet, dass** man die Behandlung des Triethanolamins mit dem Zusatz bei einer Temperatur im Bereich von 40 bis 250°C durchführt.

8. Verfahren nach einem der vorhergehenden Ansprüche, **dadurch gekennzeichnet, dass** man dem Triethanolamin 0,001 bis 2 Gew.-%, bezogen auf das reine Triethanolamin, des Phosphans und/oder der Verbindung, die ein Phosphan freisetzt, zusetzt.

9. Verfahren nach einem der Ansprüche 3 bis 8, **dadurch gekennzeichnet, dass** es sich bei dem Phosphid um ein Phosphid eines Metalls der Gruppen IA, IIA, IIIA oder IIB des Periodensystems handelt.

10. Verfahren nach einem der Ansprüche 3 bis 9, **dadurch gekennzeichnet, dass** es sich bei dem Phosphid um Lithiumphosphid, Natriumphosphid, Kaliumphosphid, Magnesiumphosphid, Calciumphosphid, Aluminiumphosphid, Indiumphosphid und/oder Zinkphosphid handelt.

11. Verfahren nach einem der vorhergehenden Ansprüche, **dadurch gekennzeichnet, dass** das Triethanolamin Wasser enthält.

12. Verfahren nach einem der Ansprüche 1 bis 10, **dadurch gekennzeichnet, dass** das Triethanolamin im Bereich von 0,01 bis 20 Gew.-% ,bezogen auf das reine Triethanolamin, Wasser enthält.

13. Verfahren nach einem der vorhergehenden Ansprüche zur Herstellung von Triethanolamin mit verbesserter Farbqualität.

14. Triethanolamin enthaltend mindestens 0,001 Gew.-% Phosphan und/oder einer Verbindung, die ein Phosphan freisetzt.

15. Triethanolamin nach dem vorhergehenden Anspruch, **dadurch gekennzeichnet, dass** es sich bei dem Phosphan um PH₃, P₂H₄ oder P₄H₆ handelt.

16. Triethanolamin nach Anspruch 14, **dadurch gekennzeichnet, dass** es sich bei der Verbindung, die ein Phosphan freisetzt, um ein Metallphosphid handelt.

17. Triethanolamin nach dem vorhergehenden Anspruch, **dadurch gekennzeichnet, dass** das Metall des Phosphids ausgewählt ist aus den Metallen der Gruppen IA, IIA, IIIA und IIB des Periodensystems.

18. Triethanolamin nach Anspruch 16, **dadurch gekennzeichnet, dass** es sich bei dem Metallphosphid um Lithiumphosphid, Natriumphosphid, Kaliumphosphid, Magnesiumphosphid, Calciumphosphid, Aluminiumphosphid, Indiumphosphid und/oder Zinkphosphid handelt.

19. Triethanolamin nach einem der Ansprüche 14 bis 18, **dadurch gekennzeichnet, dass** das Triethanolamin mindestens 0.001 Gew-% ,bezogen auf das reine Triethanolamin, des Phosphans und/oder der Verbindung, die ein Phosphan freisetzt, enthält.

20. Triethanolamin nach einem der Ansprüche 14 bis 18, **dadurch gekennzeichnet, dass** das Triethanolamin 0,001 bis 2 Gew.-% ,bezogen auf das reine Triethaholamin, des Phosphans und/oder der Verbindung, die ein Phosphan freisetzt, enthält.

21. Triethanolamin nach einem der Ansprüche 14 bis 20, **dadurch gekennzeichnet, dass** das Triethanolamin Wasser enthält.

22. Triethanolamin nach einem der Ansprüche 14 bis 21, **dadurch gekennzeichnet, dass** das Triethanolamins im Bereich von 0,01 bis 20 Gew.-% ,bezogen auf das reine Triethanolamin, Wasser enthält.

## Claims

1. A method of producing triethanolamine wherein a phosphane and/or a compound which liberates a phosphane is added to the triethanolamine.

2. The method according to claim 1, wherein the phosphane is PH₃, P₂H₄ or P₄H₆.

3. The method according to claim 1 or 2, wherein the compound which liberates a phosphane is a metal phosphide.

4. The method according to one of the preceding claims, wherein the phosphane and/or the compound which liberates a phosphane is added to the triethanolamine before and/or during distillation of the triethanolamine.

5. The method according to one of the preceding claims, wherein the triethanolamine is treated with the addition over a period of at least 5 min.

6. The method according to one of claims 1 to 4, wherein the triethanolamine is treated with the addition over a period in the range from 10 min to 50 hours.

7. The method according to one of the two preceding claims, wherein the treatment of the triethanolamine with the addition is carried out at a temperature in the range from 40 to 250°C.

8. The method according to one of the preceding claims, wherein 0.001 to 2% by weight, based on the pure triethanolamine, of the phosphane and/or of the compound which liberates a phosphane is added to the triethanolamine.

9. The method according to one of claims 3 to 8, wherein the phosphide is a phosphide of a metal of groups IA, IIA, IIIA or IIB of the Periodic Table of the Elements.

10. The method according to one of claims 3 to 9, wherein the phosphide is lithium phosphide, sodium phosphide, potassium phosphide, magnesium phosphide, calcium phosphide, aluminum phosphide, indium phosphide and/or zinc phosphide.

11. The method according to one of the preceding claims, wherein the triethanolamine comprises water.

12. The method according to one of claims 1 to 10, wherein the triethanolamine comprises water in the range from 0.01 to 20% by weight, based on the pure triethanolamine.

13. The method according to one of the preceding claims for producing triethanolamine with improved color quality.

14. A triethanolamine comprising at least 0.001% by weight of phosphane and/or of a compound which liberates a phosphane.

15. The triethanolamine according to the preceding claim, wherein the phosphane is PH₃, P₂H₄ or P₄H₆.

16. The triethanolamine according to claim 14, wherein the compound which liberates a phosphane is a metal phosphide.

17. The triethanolamine according to the preceding claim, wherein the metal of the phosphide is chosen from the metals of groups IA, IIA, IIIA and IIB of the Periodic Table of the Elements.

18. The triethanolamine according to claim 16, wherein the metal phosphide is lithium phosphide, sodium phosphide, potassium phosphide, magnesium phosphide, calcium phosphide, aluminum phosphide, indium phosphide and/or zinc phosphide.

19. The triethanolamine according to one of claims 14 to 18, wherein the triethanolamine comprises at least 0.001% by weight, based on the pure triethanolamine, of the phosphane and/or of the compound which liberates a phosphane.

20. The triethanolamine according to one of claims 14 to 18, wherein the triethanolamine comprises 0.001 to 2% by weight, based on the pure triethanolamine, of the phosphane and/or of the compound which liberates a phosphane.

21. The triethanolamine according to one of claims 14 to 20, wherein the triethanolamine comprises water.

22. The triethanolamine according to one of claims 14 to 21, wherein the triethanolamine comprises water in the range from 0.01 to 20% by weight, based on the pure triethanolamine.

## Revendications

1. Procédé pour la préparation de triéthanolamine, **caractérisé en ce qu'**on ajoute un phosphane et/ou un composé qui libère un phosphane à la triéthanolamine.

2. Procédé selon la revendication 1, **caractérisé en ce qu'**il s'agit, pour le phosphane, de PH₃, de P₂H₄ ou de P₄H₆.

3. Procédé selon les revendications 1 ou 2, **caractérisé en ce qu'**il s'agit, pour le composé qui libère un phosphane, d'un phosphure de métal.

4. Procédé selon l'une quelconque des revendications précédentes, **caractérisé en ce qu'**on ajoute le phosphane et/ou le composé qui libère un phosphane à la triéthanolamine avant et/ou pendant la distillation de la triéthanolamine.

5. Procédé selon l'une quelconque des revendications précédentes, **caractérisé en ce qu'**on traite la triéthanolamine avec l'ajout pendant un laps de temps d'au moins 5 minutes.

6. Procédé selon l'une quelconque des revendications 1 à 4, **caractérisé en ce qu'**on traite la triéthanolamine avec l'ajout pendant un laps de temps dans la plage de 10 minutes à 50 heures.

7. Procédé selon l'une quelconque des deux revendications précédentes, **caractérisé en ce qu'**on réalise le traitement de la triéthanolamine avec l'ajout à une température dans la plage de 40 à 250°C.

8. Procédé selon l'une quelconque des revendications précédentes, **caractérisé en ce qu'**on ajoute 0,001 à 2% en poids, par rapport à la triéthanolamine pure, du phosphane et/ou du composé qui libère un phosphane à la triéthanolamine.

9. Procédé selon l'une quelconque des revendications 3 à 8, **caractérisé en ce qu'**il s'agit, pour le phosphure, d'un phosphure d'un métal des groupes IA, IIA, IIIA ou IIB du système périodique.

10. Procédé selon l'une quelconque des revendications 3 à 9, **caractérisé en ce qu'**il s'agit, pour le phosphure, de phosphure de lithium, de phosphure de sodium, de phosphure de potassium, de phosphure de magnésium, de phosphure de calcium, de phosphure d'aluminium, de phosphure d'indium et/ou de phosphure de zinc.

11. Procédé selon l'une quelconque des revendications précédentes, **caractérisé en ce que** la triéthanolamine contient de l'eau.

12. Procédé selon l'une quelconque des revendications 1 à 10, **caractérisé en ce que** la triéthanolamine contient de l'eau dans la plage de 0,01 à 20% en poids, par rapport à la triéthanolamine pure.

13. Procédé selon l'une quelconque des revendications précédentes pour la préparation de triéthanolamine avec une qualité de teinture améliorée.

14. Triéthanolamine contenant au moins 0,001% en poids de phosphane et/ou d'un composé qui libère un phosphane.

15. Triéthanolamine selon la revendication précédente, **caractérisée en ce qu'**il s'agit, pour le phosphane, de PH₃, de P₂H₄ ou de P₄H₆.

16. Triéthanolamine selon la revendication 14, **caractérisée en ce qu'**il s'agit, pour le composé qui libère un phosphane, de phosphure de métal.

17. Triéthanolamine selon la revendication précédente, **caractérisée en ce que** le métal du phosphure est choisi parmi les métaux des groupes IA, IIA, IIIA et IIB du système périodique.

18. Triéthanolamine selon la revendication 16, **caractérisée en ce qu'**il s'agit, pour le phosphure de métal, de phosphure de lithium, de phosphure de sodium, de phosphure de potassium, de phosphure de magnésium, de phosphure de calcium, de phosphure d'aluminium, de phosphure d'indium et/ou de phosphure de zinc.

19. Triéthanolamine selon l'une quelconque des revendications 14 à 18, **caractérisée en ce qu'**on ajoute au moins 0,001% en poids, par rapport à la triéthanolamine pure, du phosphane et/ou du composé qui libère un phosphane à la triéthanolamine.

20. Triéthanolamine selon l'une quelconque des revendications 14 à 18, **caractérisée en ce qu'**on ajoute 0,001 à 2% en poids, par rapport à la triéthanolamine pure, du phosphane et/ou du composé qui libère un phosphane à la triéthanolamine.

21. Triéthanolamine selon l'une quelconque des revendications 14 à 20, **caractérisée en ce que** la triéthanolamine contient de l'eau.

22. Triéthanolamine selon l'une quelconque des revendications 14 à 21, **caractérisée en ce que** la triéthanolamine contient de l'eau dans la plage de 0,01 à 20% en poids, par rapport à la triéthanolamine pure.
